# EUROPEAN PATENT APPLICATION

(11) **EP 1 216 713 A1**
(43) Date of publication of application: **26.06.2002**
(21) Application number: 00610135.6
(22) Date of filing: 20.12.2000
(51) Int. Cl.: A61K 47/48, A61K 31/565

(54) **Compositions of estrogen-cyclodextrin complexes**

(71) Applicant: SCHERING AKTIENGESELLSCHAFT, 13342 Berlin (DE)
(72) Inventor: Backensfeld, Thomas, 13127 Berlin (DE); Heil, Wolfgang, 10825 Berlin (DE)
(74) Representative: Plougmann, Vingtoft & Partners A/S

(57) **Abstract**

Clathrates between cyclodextrin and an estrogen in pharmaceutical compositions conferred an increased stability to said estrogen. The estrogen ethinyl estradiol had an increased resistance to oxidative degradation when part of the inclusion complex as measured at an array of temperatures and relative humidity levels. Compositions formulated to limit the amount of oxidants also increased the stability of the estrogen. Pharmaceutical compositions comprising an estrogen for female contraception, hormone replacement therapy, menopause, or acne have longer storage lives and may require less amounts of the active substance.

## Description

### FIELD OF INVENTION

The present invention relates to pharmaceutical composition comprising a cyclodextrin-estrogen inclusion complex that confers very high chemical stability to the estrogen. The composition according to the present invention allows for an increased stability of ethinyl estradiol upon storage.

### BACKGROUND OF THE INVENTION

Degradation of estrogens, such as ethinyl estradiol, in conventional formulations is one of the most critical issues with regard to product shelf life. The stabilisation of the estrogen may be achieved by either product packaging in hermetic containers or, more effectively, as in the present invention, by actual stabilisation of the formulation.

Formulations comprising naturally or synthetically derived sex hormones often consist of low dosages of these active ingredients. Given the small amounts of active ingredient required per single dosage, often ranging between 0.1 µg and 500 µg, content uniformity of the preparation is a significant contributor to fluctuations in actual doses of these active ingredients.

Similarly, the oxidative degradation of these small amounts of active ingredient is a further contributor to the strong fluctuations of the active ingredient in low dosage formulations. In low dosage formulations, these fluctuations are critical to quality assurance.

In general, these low dose formulations are problematic in terms of their preparation, storage and use.

US 4,596,795 discloses a complex between α-, β- and γ-cyclodextrins and derivatives thereof with testosterone, progesterone, and estradiol and the solubility of said complexes.

US 4,727,064 discloses a method of producing a stabilizing amorphous complex of a drug and a mixture of cyclodextrin derivatives.

US 4,383,992 discloses a water soluble inclusion compound formed by complexing beta-cyclodextrin with a steroid compound, such an estogen.

US 5,798,338 discloses a composition of a clathrate between β-cyclodextrin and 17-α-ethinyl estradiol for reducing the oxidative degradation of 17-α-ethinyl estradiol.

### SUMMARY OF THE INVENTION

An object of the present invention is to provide a pharmaceutical composition comprising an estrogen wherein the stability of said estrogen is significantly improved over that of conventional formulations without complexed estrogens. Degradation of estrogens, such as ethinyl estradiol, in conventional formulations is one of the most critical issue with regard to product shelf life. The stabilisation of the estrogen may be achieved by either product packaging in hermetic containers (e.g. pouch) or, more effectively, by actual stabilisation of the formulation.

It has surprisingly been found that a complex of cyclodextrin and estrogen significantly improves the stability of the estrogen and consequently the shelf-life of an estrogen-containing composition. Accordingly, the present invention relates to a pharmaceutical composition comprising an inclusion complex between cyclodextrin, or derivative thereof, and an estrogen, wherein the composition is such that said estrogen has a 12-month stability at 40°C and 75% relative humidity (RH) corresponding to a decrease in the content of said estrogen of at the most 10% w/w, such as at the most 5, 4, 3, or 2% w/w, based on the total content of said estrogen, together with one or more pharmaceutically acceptable carriers or excipients.

Moreover, an alternative embodiment of the present invention relates to a pharmaceutical composition comprising an inclusion complex between cyclodextrin, or derivative thereof, and an estrogen, wherein the composition is such that said estrogen has a 12-month stability at 25°C and 60% relative humidity (RH) corresponding to a decrease in the content of said estrogen of at the most 10% w/w, such as at the most 5, 4, 3 or 2% w/w, based on the total content of said estrogen, together with one or more pharmaceutically acceptable carriers or excipients.

The present invention further relates to a pharmaceutical composition comprising an inclusion complex between cyclodextrin, or derivative thereof, and estrogen wherein the composition is such that said estrogen has a 12-month stability at 40°C and 75% relative humidity (RH) corresponding to a decrease in the content of said estrogen of at the most 10%, such as at the most 5, 4, 3 or 2% based on the total content of estrogen, and a therapeutically active compound, such as a gestagen, such as drospirenone, together with one or more pharmaceutically acceptable carriers or excipients.

Furthermore, the invention relates to a pharmaceutical composition comprising an inclusion complex between a cyclodextrin, or derivative thereof, and an estrogen, suitably formulated such that the total amount of oxidant is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen, together with one or more pharmaceutically acceptable carriers or excipients.

Compositions of the present invention may be used as medicaments. Accordingly, the use of a composition described infra for the preparation of a pharmaceutical for female contraception, for hormone replacement therapy, for the treatment of menopausal, pre-menopausal, and/or post-menopausal symptoms, for the treatment of acne or for the treatment of PMDD is a further aspect of the present invetion.

As the formulation of the complex is another important aspect to achieve the improved effects, the present invention further relates to a pharmaceutical particulate formulation of a composition, said composition comprising an inclusion complex between a cyclodextrin, or derivative thereof, wherein the total amount of oxidant in said formulation is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen.

Alternatively measured, the present invention provides a pharmaceutical particulate formulation of a composition, said composition comprising an inclusion complex between a cyclodextrin, or derivative thereof, wherein the total amount of oxidant in said formulation is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen.

Still further, the present invention relates to pharmaceutical particulate formulation of a composition, said composition comprising an inclusion complex between a cyclodextrin, or derivative thereof, wherein the total amount of oxidant in said formulation is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen.

In a broad sense, the present invention relates a method of improving the stability of ethinyl estradiol in a pharmaceutical formulation comprising the step of a) complexing said ethinyl estradiol with cyclodextrin, and optionally of b) limiting the polyvinylpyrrolidone content to less than 5%, such as less than 4%, such as less than 3%, such as less than 2%, such as less than 1%, such as providing a substantially polyvinylpyrrolidone-free formulation.

Generally, an object of the present invention is to provide a pharmaceutical composition comprising an estrogen wherein the stability of said estrogen is significantly improved over that of conventional formulations.

### DETAILED DESCIPTION OF THE INVENTION

The term "complex" is intended to mean an inclusion complex between estrogen and cyclodextrin, wherein a molecule of said estrogen is at least partially inserted into the cavity of one or more cyclodextrin molecules, wherein for example two moieties of a single estrogen molecule are each inserted into one cyclodextrin molecule to give 2:1 ratio between cyclodextrin and estrogen. Similarly, the complex may comprise of more than one molecule of estrogen at least partially inserted into one or more cyclodextrin molecules, wherein for example 2 estrogen molecules are at least partially inserted into a single cyclodextrin molecule, to give a 1:2 ratio between cyclodextrin and estrogen. Complexes wherein one estrogen molecule is complexed with one or more cyclodextrin molecules are certainly anticipated such as 1 estrogen molecule complexed with 2 cyclodextrin molecules or 3 cyclodextrin molecules. Typically, the ethinyl estradiol-β-cyclodextrin inclusion complex as prepared by the present invention is preferably a complex between one molecule of ethinyl estradiol and two molecules of β-Cyclodextrin.

The term "ethinyl estradiol-β-Cyclodextrin inclusion complex" or "EE-β-CD" is intended to mean a complex, of any ratio, between ethinyl estradiol and β-cyclodextrin.

The term "oxidant-free" relates to formulations substantially devoid of all agents having substantial oxidizing capacity whereas the term "oxidant-restricted" relates to formulations substantially devoid of at least one agent having substantial oxidizing capacity. For example, a polyvinylpyrrolidone-free formulation is an oxidant-restricted formulation.

The present investigators have developed formulations wherein a remarkable improvement of the stability of the estrogen ethinyl estradiol (EE) has been achieved by individual or combined means. One such means is the protection of EE by forming a β-cyclodextrin complex. Another such means is by the judicious selection of additives and excipients to the formulation such that oxidants are minimised or excluded from the formulation. Individually, or acting in concert, these means have resulted in formulations wherein said estrogen has a 12-month stability at 40°C and 75% relative humidity (RH) corresponding to a decrease in the content of said estrogen of between 3 and 10 % w/w, compared to conventional formulations wherein estrogen degrades by up to 25% under identical conditions (see Table 1.3, Example 1). Thus, a preferred embodiment of the invention is that of a pharmaceutical composition comprising an inclusion complex between cyclodextrin, or derivative thereof, and an estrogen, wherein the composition is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen, together with one or more pharmaceutically acceptable carriers or excipients.

It is evident that the stability test may be performed under alternative conditions such as higher temperatures. Thus an alternative embodiment wherein the composition is such that said estrogen has a 3-month stability at 60°C and 75% relative humidity (RH) corresponding to a decrease in content of said estrogen of at the most 15% w/w, such as at the most 10, 9, 8, 7, 6, or 5% w/w, based on the total content of said estrogen.

As stated, the formulation of the estrogen-cyclodextrin complex is critical to achieve the desired effect of improved stability of a composition comprising said complex. The present inventors have developed a pharmaceutical composition comprising an inclusion complex between a cyclodextrin, or derivative thereof, and an estrogen, suitably formulated such that the total amount of oxidant is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen, together with one or more pharmaceutically acceptable carriers or excipients.

An embodiment of an oxidant-restricted formulation (see Table 1.3, Example 1) comprising EE complexed with βCD compares surprisingly well with known formulations in 3-month stability studies at 60°C and 75% relative humidity (RH), wherein a less than 4% decrease in EE content was observed in said embodiment compared to an almost 30% decrease was observed in known formulations or free-estrogen formulations.

As the person skilled in the art will appreciate, the estrogen may be selected from the group consisting of ethinyl estradiol (EE), estradiol, estradiol sulfamates, estradiol valerate, estradiol benzoate, estrone, estriol, estriol succinate and conjugated estrogens, including conjugated equine estrogens such as estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequilenin sulfate and17α-dihydroequilenin sulfate or mixtures thereof. Particularly interesting estrogens are selected from the group consisting of ethinyl estradiol (EE), estradiol, estradiol sulfamates, estradiol valerate, estradiol benzoate, estrone, and estrone sulfate or mixtures thereof, notably ethinyl estradiol (EE), estradiol, estradiol valerate, estradiol benzoate and estradiol sulfamates. Most preferred is ethinyl estradiol (EE).

In the preferred embodiment wherein the estrogen is ethinyl estradiol (EE), some of the degradation products are well known. Thus it is possible to express the stability of the formulation in terms of the amount of these degradation products. Accordingly, the composition according to one embodiment of the present invention comprises an inclusion complex between cyclodextrin, or derivative thereof, and ethinyl estradiol, wherein the composition is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto-ethinyl estradiol, Δ9,11-ethinyl estradiol and Δ6,7-ethinyl estradiol of said ethinyl estradiol totals at the most 0.8%, such as at the most 0.7% and 0.6%, based on the total content of estrogen, together with one or more pharmaceutically acceptable carriers or excipients.

A pharmaceutical composition comprising an inclusion complex between a cyclodextrin, or derivative thereof, and ethinyl estradiol, suitably formulated such that the total amount of oxidant is such that said estrogen has a 12-month stability at 40°C and 75% relative humidity (RH) such that the sum product of the degradation products 6-α-hydroxy-ethinyl estradiol, 6-β-hydroxy-ethinyl estradiol, 6-keto-ethinyl estradiol, Δ6,7-ethinyl estradiol and Δ-9,11-ethinyl estradiol of said ethinyl estradiol totals at the most 3%, such as at the most 2% based on the total content of estrogen, together with one or more pharmaceutically acceptable carriers or excipients.

When exposed to an alternative stability test, the composition comprises an inclusion complex between cyclodextrin, or derivative thereof, and ethinyl estradiol, wherein the composition is such that said ethinyl estradiol has a 12-month stability at 40°C and 75% relative humidity (RH) such that the sum product of the degradation products 6-α-hydroxy-ethinyl estradiol, 6-β-hydroxy-ethinyl estradiol, 6-keto- ethinyl estradiol and Δ-9,11-ethinyl estradiol of said ethinyl estradiol totals at the most 3%, such as at the most 2 % based on the total content of estrogen, together with one or more pharmaceutically acceptable carriers or excipients.

The cyclodextrin may be selected from α- cyclodextrin, β- cyclodextrin, γ- cyclodextrin and derivatives thereof. The cyclodextrin may be modified such that some or all of the primary or secondary hydroxyls of the macrocycle, or both, may be alkylated or acylated. Methods of modifying these alcohols are well known to the person skilled in the art and many are commercially available. Thus, some or all of the hydroxyls of cyclodextrin may modified cyclodextrins have be substituted with an O-R group or an O-C(O)-R, wherein R is an optionally substituted C₁₋₆ alkyl, an optionally substituted C₂₋₆ alkenyl, an optionally substituted C₂₋₆ alkynyl, an optionally substituted aryl or heteroaryl group. Thus, R may be methyl, ethyl, propyl, butyl, pentyl, or hexyl group. Consequently, O-C(O)-R may be an acetate. Furthermore, with the commonly employed 2-hydroxyethyl group, or 2-hydroxypropyl group R may be used to derivatize cyclodextrin. Moreover, the cyclodextrin alcohols may be per-benzylated, per-benzoylated, or benzylated or benzoylated on just one face of the macrocycle, or wherein only 1, 2, 3, 4, 5, or 6 hydroxyls are benzylated or benzoylated. Naturally, the cyclodextrin alcohols may be per-alkylated or per-acylated such as per-methylated or per-acetylated, or alkylated or acylated, such as methylated or acetylated, on just one face of the macrocycle, or wherein only 1, 2, 3, 4, 5, or 6 hydroxyls are alkylated or acylated, such as methylated or acetylated.
Preferably, the complex comprises of β-cyclodextrin or a derivative thereof, most preferably β-cyclodextrin.

Thus, in a particularly preferred embodiment which is a combination of preferred embodiments, the estrogen is ethinyl estradiol and the cyclodextrin is β-cyclodextrin.

The estrogen-cyclodextrin complex may be obtained by methods known to the person skilled in the art (e.g. US 5,798,338).

The ethinyl estradiol-β-cyclodextrin complex may also be obtained by co-precipitation as follows: Ethinyl estradiol is dissolved in ethanol; β-cyclodextrin is dissolved at 45°C in water; the ethinyl estradiol solution is added to the beta-cyclodextrin solution; the obtained suspension is stirred for some hours at 20 to 25°C and afterwards at 2°C; the crystallisation product is isolated and dried.

Alternatively, the ethinyl estradiol-β-cyclodextrin complex may be obtained as follows: Ethinyl estradiol is dissolved in acetone; β-cyclodextrin is dissolved at 45°C in water; the ethinyl estradiol solution is added to the β-cyclodextrin solution; the obtained suspension is stirred for some hours at temperatures below 25°C; afterwards, the crystallisation product is isolated and dried.

In an alternative embodiment of the invention, the composition further comprises a therapeutically active compound. Thus, in this embodiment, the composition comprises an inclusion complex between cyclodextrin, or derivative thereof, and estrogen wherein the composition is such that said estrogen has a 12-month stability at 40°C and 75% relative humidity (RH) corresponding to a decrease in the content of said estrogen of at the most 10%, such as at the most 5, 4, 3 or 2% based on the total content of estrogen, and a therapeutically active compound, such as a gestagen, preferably drospirenone together with one or more pharmaceutically acceptable carriers or excipients.

In the preferred embodiment wherein the therapeutically active substance is drospirenone, said drospirenone is preferably micronised. Micronization of drospirenone provides for improved dissolution in aqueous media, potentially improving bioavailability of the gestagen.

In the preferred embodiment where the therapeutically active substance is drospirenone, all or substantially all of said drospirenone may be present as an inclusion complex with cyclodextrin. As the person skilled in the art will appreciate, the dissociation constant of the drospirenone cyclodextrin complex may result in a mixture of cyclodextrin-complexed drospirenone and uncomplexed (free) drospirenone. As was the case for uncomplexed drospirenone, the complexed drospirenone may also be micronized.

The complex of EE and cyclodextrin also has a more rapid dissolution in water than EE itself, putatively improving bioavailability of the estrogen

An object of the invention is to provide a stable composition wherein said composition comprises an inclusion complex between cyclodextrin, preferably β-cyclodextrin, and ethinyl estradiol wherein the composition is such that said estrogen has a 12-month stability at 40°C and 75% relative humidity (RH) corresponding to a decrease in the content of said estrogen of at the most 10%, such as at the most 5, 4, 3 or 2% based on the total content of estrogen, and a therapeutically active compound, such as a gestagen, preferably drospirenone, together with one or more pharmaceutically acceptable carriers or excipients.

It can be easily appreciated that in embodiments where the estrogen is ethinyl estradiol, said ethinyl estradiol may be micronized. Similarly, complexes between ethinyl estradiol and cyclodextrins may be micornized.

In embodiments wherein the composition comprises the estrogen ethinyl estradiol, the dose of the ethinyl estradiol may correspond to a daily release of the said estrogen of from 0.002 to 1 mg, such as from 0.005 to 0.5 mg, such as from 0.01 to 0.25 mg, preferably from 0.01 to 0.1 mg, such as from 0.01 to 0.05 mg, from 0.01 to 0.03 mg, such as 0.02 mg.

Thus, one embodiment of the invention is a composition comprising an inclusion complex between cyclodextrin, or derivative thereof, and ethinyl estradiol having a 12-month stability at 40°C and 75% relative humidity (RH) corresponding to a decrease in the content of said ethinyl estradiol of at the most 10%, such as at the most 5, 4, 3 or 2%, based on the total content of ethinyl estradiol, wherein the dose of ethinyl estradiol corresponds to a daily release of estrogen of from 0.002 to 1 mg, such as from 0.005 to 0.5 mg, such as from 0.01 to 0.25 mg, preferably from 0.01 to 0.1 mg, such as from 0.01 to 0.05 mg, from 0.01 to 0.03 mg, such as 0.02 mg, together with one or more pharmaceutically acceptable carriers or excipients,

In embodiments wherein the composition further comprises a therapeutically active compound and that said compound is drospirenone, the dose of drospirenone may correspond to a daily release of said gestagen of from 0.1 to 10 mg, preferably from 0.2 to 8 mg, such as from 0.25 to 6 mg, preferably from 0.5 to 5 mg, such as from 1.5 to 4 mg, such as 1, 2, 3, 4 mg.

One embodiment of the invention relates to composition comprising an inclusion complex between cyclodextrin, or derivative thereof, and ethinyl estradiol having a 12-month stability at 40°C and 75% relative humidity (RH) corresponding to a decrease in the content of said ethinyl estradiol of at the most 10%, such as at the most 5, 4, 3 or 2%, based on the total content of ethinyl estradiol, and drospirenone wherein the dose of drospirenone corresponds to a daily release of said gestagen between 1 and 5 mg, such as 3 mg, together with one or more pharmaceutically acceptable carriers or excipients.

A particularly preferred embodiment comprises of a composition comprising an inclusion complex between cyclodextrin, or derivative thereof, and ethinyl estradiol having a 12-month stability at 40°C and 75% relative humidity (RH) corresponding to a decrease in the content of said ethinyl estradiol of at the most 10%, such as at the most 5, 4, 3, or 2%, based on the total content of ethinyl estradiol, wherein the dose of ethinyl estradiol corresponds to a daily release of estrogen of from 0.002 to 1 mg, such as from 0.005 to 0.5 mg, such as from 0.01 to 0.25 mg, preferably from 0.01 to 0.1 mg, such as from 0.01 to 0.05 mg, from 0.01 to 0.03 mg, such as 0.02 mg, and drospirenone and wherein the dose of drospirenone corresponds to a daily release of said gestagen between 1 and 5 mg, such as 3 mg, together with one or more pharmaceutically acceptable carriers or excipients.

As stated, an object of the present invention is to provide a pharmaceutical composition comprising an estrogen wherein the stability of said estrogen is significantly improved over that of conventional formulations. Degradation of estrogens, such as ethinyl estradiol, in conventional formulations is the most critical issue with regard to product shelf life. One means of increasing the stability of an estrogen such as ethinyl estradiol (EE) in a composition is by the judicious selection of additives and excipients to the formulation such that oxidants are minimised or excluded from the formulation.

Thus, the formulation of the compositions of the present invention is also critical. The gain stability of the estrogen by means of complexation with cyclodextrin must be maintained in the formulation. A vigilant control of the amount of oxidant used in formulation is critical to maintaining the stability of the estrogen. Consequently, the present invention relates to a pharmaceutical particulate formulation of a composition, said composition comprising an inclusion complex between a cyclodextrin, or derivative thereof, wherein the total amount of oxidant in said formulation is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen.

Again, alternatively measured, the present invention relates to a pharmaceutical particulate formulation of a composition, said composition comprising an inclusion complex between a cyclodextrin, or derivative thereof, wherein the total amount of oxidant in said formulation is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen

Furthermore, the present invention relates to a pharmaceutical particulate formulation of a composition, said composition comprising an inclusion complex between a cyclodextrin, or derivative thereof, wherein the total amount of oxidant in said formulation is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen.

Consequently, in one embodiment of the invention, the amount of oxidants is such that the decrease in estrogen content is a further 0.1% less, such as at least 0.2, 0.3, 0.4 or 0.5% less than the decrease in estrogen content in pharmaceutical formulations of compositions not comprising an inclusion complex between a cyclodextrin, or derivative thereof, and an estrogen, as measured at the same time mark under the same conditions.

The EE-cyclodextrin complex has been found by the present investigators to be sensitive to polyvinylpyrrolidone, which is typically used as a binder for fluid bed granulations. Polyvinylpyrrolidone has a deleterious effect on the stability of estrogen, whether complexed or uncomplexed, and hence on the formulations comprising estrogens.

Thus, another aspect of the invention relates to a pharmaceutical particulate formulation of a composition, said composition comprising an inclusion complex between a cyclodextrin, or derivative thereof wherein the amount of polyvinylpyrrolidone in said formulation is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen.

Again alternatively measured the invention relates to a pharmaceutical particulate formulation of a composition, said composition comprising an inclusion complex between a cyclodextrin, or derivative thereof,
wherein the amount of polyvinylpyrrolidone in said formulation is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen.

The invention further provides a pharmaceutical particulate formulation of a composition, said composition comprising an inclusion complex between a cyclodextrin, or derivative thereof, wherein the amount of polyvinylpyrrolidone in said formulation is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen.

Most preferably, a formulation of the present invention may be characterised in that it is a polyvinylpyrrolidone-free formulation.

The investigators have prepared formulations possessing remarkable stability of the estrogen ethinyl estradiol wherein the amount of polyvinylpyrrolidone has been strictly restricted. Thus, a preferred formulation is one wherein the amount of polyvinylpyrrolidone is such that the said decrease in estrogen content is at least 0.1% less, such as at least 0.2, 0.3, 0.4 or 0.5% less than the decrease in estrogen content in pharmaceutical formulations of compositions not comprising an inclusion complex between a cyclodextrin, or derivative thereof, and an estrogen, as measured at the same time mark under the same conditions.

As can be seen from the Example 1.3, the stability of EE in formulations wherein EE is present as an inclusion complex with cyclodextrin is greater than wherein EE is present as a free steroid. The present invention thus relates to the compositions comprising EE as an inclusion complex with cyclodextrin. Furthermore, the Examples illustrates that formulations of the inclusion complex not comprising polyvinylpyrrolidone have increased stability of EE. Thus, the present invention further relates to the formulations comprising EE as an inclusion complex with cyclodextrin, preferably formulated to be polyvinylpyrrolidone-free. Example 2 demonstrates that the total amount of degradation products in formulations according to the present invention is dramatically reduced in comparison to conventional formulations.

The Examples teach that oxidation of EE by oxidants is significant in reducing the stability of EE. Consequently, it is anticipated that a formulation according to the present invention may further comprise an anti-oxidant.

A further object of the invention is to provide a composition as described *infra* adapted to be formulated for conventional granulation, pelletation, dry powder blend, fluid bed granulation, encapsulation, or compactation.

A preferred embodiment of the invention provides a particulate formulation comprising a composition as described *infra.* The particulate formulation may be prepared by a method involving a fluid bed granulation procedure, or conventional granulation, pelletation, dry powder blend, encapsulation, or compactation

The formulations comprising a composition as described *infra* may be in unit dosage form and may be adapted for the preparation of solid dosage forms or dispersion forms, as well as adapted to the preparation of tablets, capsules or sachets.

A further aspect of the invention is to provide a composition as described *infra* adapted to be administered by oral, parenteral, mucosal, nasal, vaginal or topical administration.

Formulations comprising either EE or EE-cyclodextrin complex, either with or without polyvinylpyrrolidone, and either with or without drospirenone, and with varying common excipients were prepared to establish features or combination of features which improve the stability of EE.

A typical formulation may comprise (wt/wt)
i) 0.002-1% ethinyl estradiol (micronised)
ii) 10-99%, such as 45-60% lactose monohydrate
iii) 1-80%, such as 1-35% corn starch
iv) 0-90%, such as 0-35% cellulose (microcrystallised)
v) 0.05-5% magnesium stearate
vi) 0-5% colloidal silicon dioxide

In the embodiment wherein the composition further comprises a therapeutically active compound, such as a gestagen, preferably drospirenone, a typical formulation may comprise (wt/wt)
i) 0.1-15% drospirenone (micronised)
ii) 0.002-1% ethinyl estradiol (micronised)
iii) 10-99% such as 45-60% lactose monohydrate
iv) 1-80%, such as 1-35% corn starch
v) 0-90%, such as 0-35% cellulose (microcrystallised)
vi) 0.05-5% magnesium stearate
vii) 0-5% colloidal silicon dioxide.

A preferred formulation of a tablet core consists of 1.5 to 4 mg, most preferably 3 mg of drospirenone, 0.01 to 0.03 mg, most preferably 0.02 mg of EE as an EE-βCD complex, lactose monohydrate, corn starch, magnesium stearate.

A further object of the invention is to provide a process for preparing a formulation as described *infra* the process comprising suspending maize starch in purified water, adding lactose monohydrate, ethinyl estradiol-cyclodextrin complex, optionally adding drospirenone, granulating and drying in a fluid bed granulator.

Formulations and compositions described *infra* and in the Examples are anticipated for use in the preparation of a pharmaceutical for female contraception, for hormone replacement therapy, for the treatment of menopausal, pre-menopausal, and/or post-menopausal symptoms, for the treatment of acne or for the treatment of PMDD.

A still further object of the invention is to provide a method of improving the stability of ethinyl estradiol in a pharmaceutical composition comprising the step of complexing said ethinyl estradiol with cyclodextrin. Moreover, an object of the present invention is to provide a method of improving the stability of ethinyl estradiol in a pharmaceutical formulation comprising the step of a) complexing said ethinyl estradiol with cyclodextrin, and optionally of b) limiting the polyvinylpyrrolidone content to less than 5%, such as less than 4%, such as less than 3%, such as less than 2%, such as less than 1%, such as providing a substantially polyvinylpyrrolidone-free formulation.

The present invention is further defined by the Examples herein which are not intended to be limiting in any way.

### BRIEF DESCRIPTION OF THE EXAMPLES

Example 1 discloses a composition and a formulation according to some embodiments of the present invention. The content of formulations known to the person skilled in the art are outlined in comparison with the contents of embodiments of the present invention and in comparison to a formulation which is oxidant-restricted, i.e. free of povidone. Table 1.3 illustrates the performance in terms of stability in comparison to known formulations after a fixed period of time under controlled environmental conditions. Direct compression of the powder blend resulted in a dramatic improvement in the stability of EE in comparison to blends which comprise the free steroid. The Formulation E was prepared to be povidone-free. This also results in increased stability of the active ingredient. By comparison, the fluid-bed formulation, which is also povidone-free, does not perform as well as those formulations wherein EE is complexed with cyclodextrin. Complexation of EE with cyclodextrin improves the stability of EE.

Example 2 illustrates the stability of EE in Formulations D and E in comparison to other formulations in terms of the breakdown products isolated from samples upon storage after a fixed period of time under controlled environmental conditions.

Example 3 discloses the contents of one embodiment of the present invention, wherein the composition further comprises drospirenone.

Example 4 describes the morphology or some physical characteristics of a typical dosage form of a formulation according to the present invention.

Example 5 discloses a typical process for the preparation of a tablet.

Example 6 describes the method in which certain physical properties, namely the rate constant of the dissociation constant of the inclusion complex between EE and CD, was studied. The half-life of the 1:1 complex was determined to be 155.8 s and the dissociation constant was determined to be 4.45 x 10⁻³ s⁻¹.

Example 7 describes the method in which the equilibrium stability constant (formation constant) of the inclusion complex between EE and CD was studied. The stability constant of the 1:1 complex was found to be 9.5 x 10⁻⁴ M⁻¹. The solubility of complexed ethinyl estradiol was found to improve in comparison to the free steroid.

Example 8 describes the method in which the equilibrium stability constant (formation constant) of the inclusion complex between EE and CD in acidic medium was studied. The stability constant of the 1:1 and 1:2 complex in acidic medium is disclosed. The solubility of complexed ethinyl estradiol was found to improve in comparison to the free steroid in acidic medium.

Example 9 discloses the method in which the acid dissociation constant (pKₐ) of the EE-CD complex in aqueous media was determined to be approx. 10.51 in comparison to the pKₐ of approx. 10.25 of the free-steroid.

Example 10 describes the method in which the n-octanol/water partition coefficient of the EE-CD inclusion complex was determined and its dependence on pH. Its log P value ranges from 3.20 to 3.53.

Example 11 discussed whether the ethinyl estradiol-β-cyclodextrin inclusion complex can exist in multiple solid state forms and to provide test methods which can detect and distinguish between such forms.

Example 12 describes typical preparations of an EE-CD complex.

### EXAMPLES

### Example 1

### Comparative of EE Content After Storage of Test Formulations

### 1. Summary of Contents of Film-Coated Tablets

**a) Formulation A**
   EE-β-CD complex, fluid bed granulation, with polyvinylpyrrolidone (povidone)
b) **Formulation B** (a standard formulation)
   EE as free steroid, fluid bed granulation with polyvinylpyrrolidone
c) **Formulation C**
   EE as free steroid, fluid bed granulation without polyvinylpyrrolidone
d) **Formulation D**
   EE-β-CD complex, direct compression of powder blend (without povidone)
e) **Formulation E**
   EE-β-CD complex, fluid bed granulation, without polyvinylpyrrolidone

### 2. Composition of Test Formulations

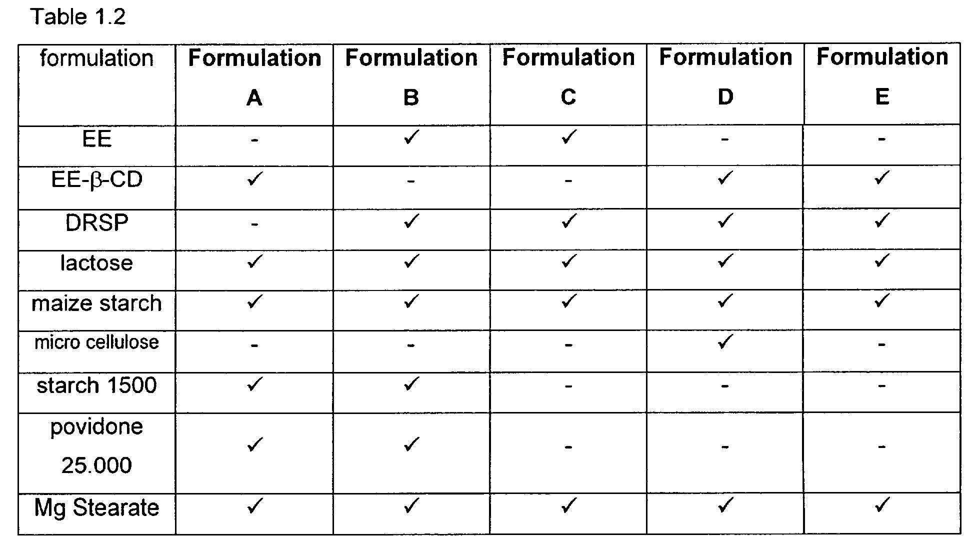

### 3. EE-Content After Storage

**Table 1.3**

| formulation | start | 3 months | | 12 months | |
|---|---|---|---|---|---|
| | | 40°C 75% RH | 60°C, 75% RH | 25°C, 60% RH | 40°C, 75% RH |
| **Formulation A** | 93.1 | 86.3 | 77.8 | 93.8 | 75.9 |
| **Formulation B** | 98.9 | 94.9 | 70.7 | 95.6 | 85.7 |
| **Formulation C** | 100.1 | 95.8 | 86.1 | 100.1 | 92.1 |
| | 99.1 | 96.2 | 86.1 | 99.1 | 92.1 |
| **Formulation D** | 101.5 | 98.8 | 96.4 | 101.4 | 99.9 |
| | 102.7 | 100.7 | 98.6 | 101.8 | 100.0 |
| **Formulation E** | 103.2 | 101.3 | **96.4** | **100.5** | **98.9** |
| | 103.3 | 102.0 | **96.6** | **101.8** | **99.3** |

### Example 2

### Comparative of Known Degradation Products After Storage

**Table 2.1;**

| *After 12 months,* 25°C, *60% RH* | | | | | |
|---|---|---|---|---|---|
| formulation | 6-α-OH-EE | 6-β-OH-EE | 6-Keto-EE | Δ9,11-EE | total known |
| **EE** | 0.004 | 0.005 | n.d | 0.38 | 0.389 |
| **EE-β-CD** | 0.002 | 0.003 | n.d | 0.38 | 0.385 |
| **Formulation B** | 0.04 | 0.07 | 0.32 | 0.74 | 1.20 |
| **Formulation C** | 0.05 | 0.09 | 0.11 | 0.73 | 1.00 |
| | 0.04 | 0.07 | 0.08 | 0.70 | 0.91 |
| **Formulation D** | 0.01 | 0.02 | n.d | 0.49 | 0.52 |
| | 0.01 | 0.01 | n.d | 0.45 | 0.47 |
| **Formulation E** | **0.03** | **0.01** | **n.d** | **0.46** | **0.50** |
| | **0.02** | **0.01** | **n.d** | **0.40** | **0.43** |
| specification | ≤ 0.5 | ≤ 0.5 | ≤ 2.0 | ≤ 1.0 | - |
| n.d = not detectable; 6-α-OH-EE = 6-α-hydroxy-ethinyl estradiol; 6-βOH-EE = 6-β-hydroxy-ethinyl estradiol; 6-Keto-EE = 6-keto- ethinyl estradiol; Δ9,11-EE = Δ9,11-ethinyl estradiol. | | | | | |

**Table 2.2;**

| *After 12 months, 40°C, 75% RH* | | | | | |
|---|---|---|---|---|---|
| formulation | 6-α-OH-EE | 6-β-OH-EE | 6-Keto-EE | Δ9,11-EE | total known |
| **EE** | 0.004 | 0.005 | n.d | 0.38 | 0.389 |
| **EE-β-CD** | 0.002 | 0.003 | n.d | 0.38 | 0.385 |
| **Formulation B** | 0.16 | 0.25 | **1.92** | **3.14** | **5.47** |
| **Formulation C** | 0.33 | 0.61 | 1.03 | 1.86 | 3.83 |
| | 0.28 | 0.54 | 0.87 | 1.59 | 3.28 |
| **Formulation D** | 0.03 | 0.09 | **0.10** | **0.79** | **1.01** |
| | 0.03 | 0.10 | **0.09** | **0.79** | **0.98** |
| **Formulation E** | 0.08 | 0.19 | **0.30** | **0.93** | **1.50** |
| | 0.08 | 0.19 | **0.41** | **0.89** | **1.58** |
| specification | ≤ 0.5 | ≤ 0.5 | ≤ 2.0 | ≤ 1.0 | - |
| n.d = not detectable; 6-α-OH-EE = 6-α-hydroxy-ethinyl estradiol; 6-β-OH-EE = 6-β-hydroxy-ethinyl estradiol; 6-Keto-EE = 6-keto- ethinyl estradiol; Δ9,11-EE = Δ9,11-ethinyl estradiol. | | | | | |

### Example 3

### Typical composition

The drug product consists of the components listed below. The batch size is determined by 200,000 - 550,000 tablets (development site) and 2.5 Mio tablets (production site), respectively. Water is used as a processing aid for the manufacture of the tablet mass (fluid bed granulation) and film-coating.

| Component | **mg** per tablet | **kg** per batch (pilot site), batch size = 550,000 tablets | **kg** per batch (production site), batch size = 2.5 mio. tablets |
|---|---|---|---|
| drospirenone, micro 15 | 3.0 | 1.650 | 7.500 |
| ethinyl estradiol-β-cyclodextrine complex, micro | 0.020 * | 0.011 * | 0.050 * |
| lactose monohydrate | 48.18 | 26.499 | 120.450 |
| maize starch | 28.0 | 15.400 | 70.000 |
| magnesium stearate | 0.8 ** | 0.440 ** | 2.000 ** |
| **tablet mass weight** | **80.0 mg** | **44.000 kg** | **200.000 kg** |
| hydroxypropylmethyl cellulose | 1.5168 | 0.83424 | 3.792 |
| talc | 0.3036 | 0.16698 | 0.759 |
| titanium dioxide | 1.1748 | 0.64614 | 2.937 |
| ferric oxide pigment, red | 0.0048 | 0.00264 | 0.012 |
| **weight of film-coat** | **3.0 mg** | **1.650 kg** | **7.500 kg** |
| **total weight** | **83.0 mg** | **45.650 kg** | **207.500 kg** |

| | | | |
|---|---|---|---|
| * : quantities given state the amount of ethinyl estradiol. | | | |

### Example 4

### Description of the dosage form

| | | |
|---|---|---|
| Core: | Shape | round, biconvex |
| | Diameter | 6 mm |
| | Curvature radius | 6 mm |
| | Height | 2.95 mm |
| | Weight | 80.0 mg |
| | Hardness | 40 N |
| | Friability (Ph.Eur. / USP) | ≤ 0.5 % |
| | Disintegration time | ≤ 10 min |
| Coated tablet: | Color | light pink |
| | Weight | 83.0 mg |

### Example 5

### Description of manufacturing process

- Weigh all ingredients.
- Prepare the granulation liquid:
   - Suspend maize starch in purified water and add this suspension to purified water while stirring.
- Prepare the tablet mass:
   - Introduce lactose, drospirenone micro 15, ethinyl estradiol-β-cyclodextrine complex micro and maize starch (portion) into the fluid bed granulator. Activate a continuous fluid bed. Dry. Check relative humidity of the tablet mass. Dry the tablet mass if necessary until the desired range of relative humidity is reached.
- Check yield.
- Compress the tablet mass
   - Perform on a rotary tableting machine to tablet cores. Pass the cores through a tablet deduster.
- Prepare the film-coating suspension:
   - Suspend talc, ferric oxide pigment red and titanium dioxide in purified water and homogenise the suspension. Dissolve hydroxypropylmethyl cellulose in purified water while stirring. Combine and homogenise the mixture and check yield.
- Film-coating:
   - Introduce the tablet cores into a suitable coater and warm them up.
   - Spray the appropriate amount of film-coating suspension continuously on the rotating cores while drying with warm air. Polish and check weight uniformity, disintegration time and yield.
   - Equilibrate the film-coated tablets.
Check the disintegration time of at least 6 tablets

### Example 6

### Determination of Rate Constant of the Dissociation Constant of EE-β-CD complex in aqueous solution.

### Background

The ethinyl estradiol-β-Cyclodextrin inclusion complex as prepared by the present invention is preferably a clathrate complex containing one molecule ethinyl estradiol and two molecules of β-Cyclodextrin.

When dissolved in water, the ethinyl estradiol-β-Cyclodextrin inclusion complex dissociates into its components ethinyl estradiol (EE) and the ligand β-Cyclodextrin (CD) following law of mass action equilibria.${\text{1. EE + CD ⇔ EE·CD K}}_{\text{11}} \text{=} \frac{{\text{c}}_{\text{EECD}}}{{\text{c}}_{\text{EE}} {\text{· c}}_{\text{CD}}}$${\text{2. EE.CD + CD ⇔ EE.CD}}_{\text{2}} {\text{K}}_{\text{12}} \text{=} \frac{\text{c} {\text{}}_{{\text{EECD}}_{\text{2}}}}{{\text{c}}_{\text{EECD}} {\text{· c}}_{\text{CD}}}$

In this study, the dissociation rate of the 1:1 complex was determined using a stopped flow relaxation method with conductometric detection.

Ethinyl estradiol is practically undissociated at pH = 7 (pKa = 10.25). For this reason it remains uncharged in aqueous solution and its contribution to the conductivity is negligible. Therefore a direct determination of the dissociation rate with the conductometric detector was not possible.

Therefore an indirect method was applied based on a competition reaction using sodiumdodecylsulfate (SDS) which forms an inclusion complex as well. SDS as a salt is dissociated in aqueous solution and contributes sufficiently to conductivity. When the SD⁻ anion binds to β-Cyclodextrin, the inclusion complex formed will be less mobile as the free SD⁻ ion in water and the electrical conductivity of the solution will decrease.

The difference in conductivity of the free DS⁻ anion and the complexed anion was used to detect the release kinetic of ethinyl estradiol from the clathrate complex with a stopped flow kinetic apparatus with conductivity detector.

As complexation of SDS with β-Cyclodextrin is very rapid, the dissociation of the EE-β-CD complex becomes rate determining and can be measured by stopped flow experiments.

At higher β-Cyclodextrin concentrations higher ordered Ethinyl estradiol-β-Cyclodextrin complexes are formed. Therefore the dissociation rate of the 1:2 complex could not be determined.

### Summary of Results

The dissociation rate constant of the Ethinyl estradiol-β-Cyclodextrin 1:1 complex was determined through stopped-flow conductometric studies of the competition reaction between sodiumdodecylsulfate and Ethinyl estradiol to form inclusion complexes with β-Cyclodextrin to be:${\text{K}}_{\text{d}} {\text{= 4.45 · 10}}^{\text{-3}} {\text{s}}^{\text{-1}}$

Under first order conditions the half live of dissociation of the Ethinyl estradiol-β-Cyclodextrin 1:1 complex was calculated to be:${\text{t}}_{\text{1/2}} \text{= 155.8 s (2.6 min)}$

### Example 7

### Determination of the Equilibrium Stability Constant (Formation Constant) of the EE-β-CD complex in aqueous solution.

### Background

The drug substance ethinyl estradiol-β-cyclodextrin inclusion complex is a clathrate complex containing one molecule Ethinyl estradiol and two molecules of β-cyclodextrin. The formation of the ethinyl estradiol-β-cyclodextrin clathrate in aqueous solution from its components ethinyl estradiol (S) and the ligand β-cyclodextrin (L) are defined by the following equations according to the law of mass action.${\text{1. S+L=SL K}}_{\text{11}} \text{=} \frac{{\text{C}}_{\text{SL}}}{{\text{C}}_{\text{S}} {\text{· C}}_{\text{L}}}$${\text{2. SL + L = SL}}_{\text{2}} {\text{K}}_{\text{12}} \text{=} \frac{\text{c} {\text{}}_{{\text{SL}}_{\text{2}}}}{{\text{c}}_{\text{SL}} {\text{· c}}_{\text{L}}}$

The equilibrium stability constant (formation constants) K₁₁ was determined with a phase solubility technique. For K₁₂ only a rough estimation was obtained.

### Summary of Results

The following data were obtained with the phase solubility diagram technique (PSD) in aqueous solutions at 20 °C.

| | |
|---|---|
| Stabiliy constant of the 1:1 complex | K₁₁ = 9.5 · 10⁴ M⁻¹ |
| Solubility of Ethinyl estradiol | S_{EE} = 2.17 · 10⁻⁵ mol/l (6.43 · 10⁻³ g/l) |
| Solubility of the 1:1 complex | S₁₁ = 1.92 · 10⁻³ mol/l (2.75 g/l) |
| Solubility of the 1:2 complex | S₁₂ = 1.44 · 10⁻³ mol/l (3.7 g/l) |

### Example 8

### Determination of the Equilibrium Stability Constant (Formation Constant) of the EE-β-CD complex in 0.1 M HCI.

### Background

The drug substance ethinyl estradiol-β-cyclodextrin complex is an inclusion complex complex containing one molecule of ethinyl estradiol and two molecules of β-cyclodextrin.

The formation of the ethinyl estradiol-β-cyclodextrin inclusion complex in aqueous solution from its components ethinyl estradiol (EE) and the ligand β-cyclodextrin (βCD) are defined by the following equations according to the law of mass action.${\text{1. EE + βCD = EEβCD K}}_{\text{11}} \text{=} \frac{{\text{c}}_{\text{EEβCD}}}{{\text{c}}_{\text{βCD}} {\text{· c}}_{\text{EE}}}$${\text{2. EEβCD + βCD = EEβCD}}_{\text{2}} {\text{K}}_{\text{12}} \text{=} \frac{\text{c} {\text{}}_{{\text{EEβCD}}_{\text{2}}}}{{\text{c}}_{\text{EEβCD}} {\text{· c}}_{\text{βCD}}}$ cᵢ = equilibrium concentrations of the respective entities (mol/l)

### Summary of Results

The following data were obtained with the phase solubility diagram technique (PSD) in 0.1 M HCI at 20 °C.

| | |
|---|---|
| Stability constant of the 1:1 complex | K₁₁ = 1.56 · 10⁴ M⁻¹ |
| Overall stability constant of the 1:2 complex (= K₁₁ · K₁₂) | K*₁₂ = approx. 1.6 · 10⁴ M⁻¹ |
| Solubility of ethinyl estradiol | S_{EE} = 1.68 · 10⁻⁴ mol/l (0.05 g/l) |
| Solubility of the 1:1 complex | S₁₁ = 2 · 10⁻³ mol/l (2.9 g/l) |
| Solubility of the 1:2 complex | S₁₂ = 5 · 10⁻⁴ mol/l (1.3 g/l) |

### Example 9

### Determination of the Acid Dissociation Constant of the EE-β-CD complex in aqueous media

### Background

The drug substance ethinyl estradiol-β-cyclodextrin inclusion complex is a clathrate complex containing one molecule of ethinyl estradiol and two molecules of β-cyclodextrin.

In aqueous solution, the ethinyl estradiol-β-cyclodextrin inclusion complex dissociates into its components according to the law of mass action. To restrain the dissociation of the ethinyl estradiol-β-cyclodextrin inclusion complex an aqueous solution containing an approx. 300 fold (0.0114 molar) excess of β-cyclodextrin over ethinyl estradiol was used in the measurements.

The pKa was measured by a photometric titration method following the guidance given in the Environmental Assessment Technical Handbook.

### Summary of Results

The acid dissociation constant of the ethinyl estradiol-β-cyclodextrin inclusion complex was determined at 20 C in aqueous solutions containing mixtures of ethinyl estradiol with an approx. 300 fold excess of β-cyclodextrin with a photometric titration method as:${\text{pK}}_{\text{a}} \text{= 10.51 ± 0.03}$

For comparison the pKa of ethinyl estradiol in the absence of β-cyclodextrin is${\text{pK}}_{\text{a}} \text{= 10.25 ± 0.04}$

### Example 10

### Determination of the n-octanol/water partition coefficient

### Background

The drug substance ethinyl estradiol-β-cyclodextrin inclusion complex is a complex containing one molecule of ethinyl estradiol and two of molecules β-cyclodextrin.

If the ethinyl estradiol-β-cyclodextrin inclusion complex is equilibrated in the two-phase system, n-octanol/water, a number of simultaneously occuring equilibria are involved: partitioning of the complex and the free components between the aqueous phase and n-octanol and dissociation of the inclusion complex. The dissociation equilibria will be shifted after phase separation for analysis of both phases. Therefore only the total amount of ethinyl estradiol in the aqueous and the octanolic phase can be determined. The result is the apparent n-octanol/water partition coefficient of ethinyl estradiol.

For determination of the pH dependence of the apparent n-octanol/water partition coefficient of ethinyl estradiol measurements were performed at pH 5, 7 and 9 with the flask-shaking method according to OECD guideline 107¹⁾.

### Summary of Results

The flask-shaking method was used to determine the apparent partition coefficient of ethinyl estradiol after partitioning the ethinyl estradiol-β-cyclodextrin inclusion complex between aqueous buffer solutions and n-octanol. Measurements were performed with aqueous solutions buffered to pH 5, 7 and 9. The ethinyl estradiol concentration in each phase after equilibration at 25 °C was determined by HPLC.

**Table 1:**

| The pH dependence of the apparent partition coefficient of ethinyl estradiol | | | |
|---|---|---|---|
| pH | mean app. P_{ow} with standard deviations | app. log P_{ow} | 95 % confidence intervals for app. log P_{ow} |
| 5 | 2395 ± 623 | 3.38 | 3.28 - 3.46 |
| 7 | 3424 ± 1298 | 3.53 | 3.35 - 3.67 |
| 9 | 1579 ± 505 | 3.20 | 3.08 - 3.29 |

### Example 11

### Determination of whether the compound consisting of the ethinyl estradiol-β-cyclodextrin inclusion complex can exist in multiple solid state forms and to provide test methods which can detect and distinguish between such forms

### Background

In order to establish whether the compound consisting of the ethinyl estradiol-β-cyclodextrin inclusion complex exists in different solid state forms a variety of crystallization products obtained under different crystallization, drying and storage conditions are investigated with respect to their solid state form. A selection of the following analytical methods is applied in order to identify and characterise solid state forms as deemed appropriate and possible:
X-ray powder diffraction (XRPD)
differential thermal analysis (DTA) in combination with thermogravimetry (TG)
differential scanning calorimetry (DSC) in combination with thermogravimetry (TG)

### Summary of Results

Evidences of complex formation is obtained by investigation of pure ethinyl estradiol and beta-cyclodextrin, mechanical mixtures of both substances as well as samples of the ethinyl estradiol-beta-cyclodextrin complex with X-ray powder diffraction and thermal analysis. According to these investigations at least about 90 % of the ethinyl estradiol should be bonded in the complex.

The prevalent form of the ethinyl estradiol-beta-cyclodextrin inclusion complex is that of a hydrate containing varying amounts of water. The variability of the water content is a consequence of an inherent property of free cyclodextrin as well as of its inclusion compounds (inclusion complexes or clathrates), the equilibration of at least part of the hydrate water with the ambient atmosphere. On storage, an equilibrium water content is established which depends on temperature, pressure and relative humidity. The hydrate water can be readily lost from the crystal lattice. Under more servere drying conditions all crystal water can be removed, however the resulting material is extremely hygroscopic and therefore not of relevance for the drug substance. The same applies to a fully water-saturated hydrate which is stable only in the presence of mother liquid or under relative humidity of more than 97 %. Thus, any discussion on the solid state forms of the ethinyl estradiol-β-cyclodextrin complex has to concentrate on the characterisation of a range of hydrates having intermediate water contents with an upper limit at water saturation.

The hydrate water is a part of the crystal lattice and therefore alterations of the water content are connected with changes in the crystal lattice. This is manifested by differences in the X-ray powder pattern of batches of clathrate with varying water content. According to these patterns four different types can be distinguished. Batches of type I contain less than 1 % water. In batches of types II and III between 4 % and 10 % water, and 8 % and 15 % water, respectively, are found. Type IV is characterised by a water content of more than 15 %. However, there's no clear dividing line between two neighbouring types. The position of the diffraction peaks is changed gradual due to swelling and shrinking of the crystal lattice during water sorption or desorption. Investigations of the four types by differential thermal analysis in combination with thermogravimetry have shown that the dehydration takes place between 25°C and 170°C. The different forms are readily and reversibly interchanged by adjustment of the ambient humidity conditions. This behaviour indicates considerable rigidity of the structural framework which does not allow profound alteration of the basic arrangement of the beta-cyclodextrin/ethinyl estradiol building blocks of the solid during hydration and dehydration.

### Example 12

### Preparation of the ethinyl estradiol-beta-cyclodextrin complex

The ethinyl estradiol-beta-cyclodextrin complex is obtained by co-precipitation as follows:
Process 1 (P1): Ethinyl estradiol is dissolved in ethanol. β-cyclodextrin is dissolved at 45 °C in water. The ethinyl estradiol solution is added to the β-cyclodextrin solution. The obtained suspension is stirred for some hours at 20 to 25°C and afterwards at 2 °C. The crystallization product is isolated and dried by methods described *infra.*
Process 2 (P2): Ethinyl estradiol is dissolved in acetone. β-cyclodextrin is dissolved at 45 C in water. The ethinyl estradiol solution is added to the beta-cyclodextrin solution. The obtained suspension is stirred for some hours at temperatures below 25 °C. Afterwards, the crystallization product is isolated and dried by methods described *infra.*

The mechanical mixtures of β-cyclodextrin and ethinyl estradiol are prepared by weighing and afterwards homogenization by grinding in an agate mortar.

**Table 12.1 a:**

| Crystallization products of the complex | | | |
|---|---|---|---|
| batch | solvent / treatment conditions | content of EE [%] | water content [%] |
| Im2180 | P 1, dried ir a vacuum drying cabinet | 10.9 | 5.57 |
| Im2181 | P 1, dried ir a vacuum drying cabinet | 11.2 | 5.26 |
| Im2182/1 | P 1, dried 1 h at r.t. over P₂O₅ in a vacuum desiccator | n.d. | 6.5 |
| Im2182/2 | P 1, dried 2 h at r.t. over P₂O₅ in a vacuum desiccator | n.d. | 6.5 |
| Im2182/3 | P 1, dried 4 h at r.t. over P₂O₅ in a vacuum desiccator | n.d. | 6.4 |
| Im2182/4 | P 1, dried 4 h at r.t. over P₂O₅ in a vacuum desiccator | n.d. | 7.7 |
| Im2182/5 | P 1, dried 43.5 h at r.t. over P₂O₅ in a vacuum desiccator | 10.8 | 4.47 |
| Im2182/6 Act. | P 1, washed with acetone, dried 3 h at 2°C over P₂O₅ in a vacuum desiccator | 10.9 | 4.65 |
| Im2182/7 | P 1, washed with acetone, and water, dried 3 h at 2°C over P₂O₅ in a vacuum desiccator | 10.6 | 4.47 |
| Im2183/V | P 1, dried some hours at r.t. over P₂O₅ in a vacuum desiccator | 11.4 | 4.21 |
| Im2183/VT | P 1, dried in a vacuum drying cabinet | 10.7 | 5.59 |
| Im2183/L | P1, stored at air | 11.4 | 10.2 |
| Im2183/VT+L | P 1, dried in a vacuum drying cabinet and than storage at air | 10.6 | 8.75 |
| Im2184 | P 1, dried in a vacuum drying cabinet | 10.9 | 5.60 |
| Im2188 | P 1, 20 h at r.t. | 10.8 | 11.85 |
| Im2190f. | P 1, dried in a vacuum drying cabinet - 1d | n.d. | - |
| Im2191f. | P 1, dried in a vacuum drying cabinet - 1d | n.d. | - |
| Im2190 | P 1, dried in a vacuum drying cabinet - 5d | 10.6 | 7.5 |
| Im2191 | P 1, dried in a vacuum drying cabinet - 5d | 10.6 | 7.7 |
| 28052591 | batch Im2190 micronized | 10.7 | 8.23 |
| Im2220 | P 2, dried in a vacuum drying cabinet | 10.7 | 5.61 |
| Im2221 | P 1, dried in a vacuum drying cabinet | 10.2 | 5.78 |
| Im2222 | P 1, dried in a vacuum drying cabinet | 10.4 | 5.57 |
| Im2223 | P 1, dried in a vacuum drying cabinet | 10.1 | 5.64 |
| Im2224 | P 2, dried in a vacuum drying cabinet | 10.4 | 5.75 |

**Table 12.1b:**

| Crystallization products of the complex | | | |
|---|---|---|---|
| batch | solvent / treatment conditions | content of EE [%] | water content [%] |
| Im2225/1 | P 1; washed 2 × water; dried in a vacuum drying cabinet | 11.2 | 3.34 |
| Im2225/2 | P 1; washed 2 × water, 1 × acetone; dried in a vacuum drying cabinet | 10.5 | 3.31 |
| Im2225/3 | P 1; washed 2 × water, 1 × acetone,1 × water; dried in a vacuum drying cabinet | 10.9 | 3.8 |
| Im2230 | P 1; washed 1 × water, 1 × acetone,1 × water; dried in a vacuum drying cabinet | 10.8 | 4.35 |
| Im2231 | P 1; washed 1 × water, 1 × acetone,1 × water; dried in a vacuum drying cabinet | 11 | 2.63 |
| Im2240 | P 1; washed 1 × water, 1 × acetone,1 × water; dried in a vacuum drying cabinet | 10.5 | 6.71 |
| 28052591,DVS1 0%RH | batch 28052591 after one sorption/desorption cycle, stored at 0 % RH | n.d. | < 1%⁵ |
| Im2180,DVS1 0% RH | batch Im2180 after sorption/desorption cycle, stored at 0 % RH | n.d. | < 1%⁵ |
| Im2180,DVS1 45% RH | batch Im2180 stored at 45 % RH | n.d. | 6.5⁵ |
| Im2180,DVS1 70% RH | batch Im2180 stored at 70 % RH | n.d. | 9.5⁵ |
| Im2180,DVS1 75% RH | batch Im2180 stored at 75 % RH | n.d. | 9.5⁵ |
| Im2180,DVS1 93% RH | batch Im2180 stored at 93 % RH | n.d. | ∼15⁵ |
| Im2180,3d Mg(ClO₄)₂ | batch Im2180 stored 3 d over Mg(ClO₄)₂ | n.d. | n.d. |
| Im2190,5d 97% RH | batch Im2190 stored 5 d at 97 % RH | n.d. | ∼16.7⁵ |
| Im2190,7d 97% RH | batch Im2190 stored 7 d at 97 % RH | n.d. | ∼16.5⁵ |
| 28052591,7d Mg(ClO₄)₂ | batch 28052591 stored 7 d over Mg(ClO₄)₂ | n.d. | < 0.1⁵ |
| 28052591,7d 97% RH | batch 28052591 stored 7 d at 97 % RH | n.d. | 16.9⁵ |
| 28052591, wet | batch 28052591 suspended in water, no drying | - | - |
| 28052591,7d 75% RH | batch 28052591 stores 7 d at 75 % RH | n.d. | 10.5⁵ |

| | | | |
|---|---|---|---|
| ⁵ calculated from the water content of the start ng material and the observed mass change | | | |

## Claims

1. A pharmaceutical composition comprising an inclusion complex between a cyclodextrin, or derivative thereof, and an estrogen
wherein the composition is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen,
together with one or more pharmaceutically acceptable carriers or excipients.

2. A composition comprising an inclusion complex between a cyclodextrin, or derivatives thereof, and estrogen
wherein the composition is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen,
together with one or more pharmaceutically acceptable carriers or excipients.

3. A pharmaceutical composition comprising an inclusion complex between a cyclodextrin, or derivative thereof, and an estrogen
suitably formulated such that the total amount of oxidant is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen,
together with one or more pharmaceutically acceptable carriers or excipients.

4. A composition according to any of c laims 1 to 3, wherein the estrogen is selected from the group consisting of ethinyl estradiol (EE), estradiol, estradiol sulfamates, estradiol valerate, estradiol benzoate, estrone, and estrone sulfate or mixtures thereof, preferably ethinyl estradiol (EE), estradiol, estrac iol valerate, estradiol benzoate and estradiol sulfamates, most preferably ethinyl estradiol (EE).

5. A composition according to claim 3, wherein the estrogen is ethinyl estradiol.

6. A composition comprising an inclus on complex between a cyclodextrin, or derivative thereof, and ethinyl estradiol,
wherein the composition is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen,
together with one or more pharmaceutically acceptable carriers or excipients.

7. A composition comprising an inclus on complex between a cyclodextrin, or derivative thereof, and ethinyl estradiol,
wherein the composition is such that said ethinyl estradiol has a 12-month stability at 40°C and 75% relative humidity (RH) such that the sum product of the degradation products 6-α-hydroxy-ethinyl estradiol 6-β-hydroxy-ethinyl estradiol, 6-keto-ethinyl estradiol, Δ6,7-ethinyl estradiol and Δ-9,11-ethinyl estradiol of said ethinyl estradiol totals at the most 3%, such as at the most 2% based on the total content of estrogen,
together with one or more pharmaceutically acceptable carriers or excipients.

8. A pharmaceutical composition comprising an inclusion complex between a cyclodextrin, or derivative thereof, and an estrogen,
suitably formulated such that the total amount of oxidant is such that said estrogen has a 12-month stability at 40°C and 75% relative humidity (RH) such that the sum product of the degradation products 6-α-hydroxy-ethinyl estradiol, 6-β-hydroxy-ethinyl estradiol, 6-keto-ethinyl estradiol, Δ6,7-ethinyl estradiol and Δ-9,11-ethinyl estradiol of said ethinyl estradiol totals at the most 3%, such as at the most 2% based on the total content of estrogen,
together with one or more pharmaceutically acceptable carriers or excipients.

9. A composition according to any of claims 1 to 8, wherein the cyclodextrin is selected from the group consisting of α-cyclodextrin, β-cyclodextrin, γ-cyclodextrin and derivatives thereof.

10. A composition according to claim 9, wherein the cyclodextrin is β-cyclodextrin or derivatives thereof.

11. A composition according to claim 10, wherein the cyclodextrin is β-cyclodextrin.

12. A composition according to any the preceding claims, further comprising one or more therapeutically active compounds.

13. A composition comprising according to claim 12, wherein at least one of the therapeutically active compounds is a gestagen.

14. A composition according to claim 13, wherein the gestagen is selected from the group consisting of drospirenone, levonorgestrel, norgestrel, gestadene, dienogest, cyproterone acetate, norethisterone, norethisterone acetate, desorgestrel, 3-keto-desorgestrel.

15. A composition according to claim 14, wherein the gestagen is drospirenone.

16. A composition according to claim 15, wherein the drospirenone is micronised.

17. A composition according to any of claims 15 or 16, wherein all or substantially all the drospirenone is present as an inclusion complex with cyclodextrin.

18. A composition according to claim 12, wherein the estrogen is ethinyl estradiol and a therapeutically active compound is drospirenone.

19. A composition according to any of claims 5, 6, 7 or 18, wherein the ethinyl estradiol is micronised.

20. A composition according to any of the preceding claims, wherein the dose of the estrogen corresponds to a daily release of ethinyl estradiol between 0.002 and 1 mg

21. A composition according to any of claims 14 to 18, wherein the dose of drospirenone corresponds to a daily release of said gestagen of from 0.1 to 10 mg, preferably from 0.25 to 6 mg, such as from 1.5 to 4 mg.

22. A composition according to any of the preceding claims, wherein the estrogen is ethinyl estradiol and the dose of ethinyl estradiol corresponds to a daily release of said estrogen of from 0.002 to 1 mg, such as from 0.005 to 0.5 mg, such as from 0.01 to 0.25 mg, preferably from 0.01 to 0.1 mg, such as from 0.01 to 0.05 mg, from 0.01 to 0.03 mg, such as 0.02 mg.

23. A composition according to claim 22, further comprising drospirenone,
wherein the dose of drospirenone corresponds to a daily release of said gestagen between 1 and 5 mg, such as 3 mg.

24. A composition according to any of the preceding claims, adapted to be formulated by conventional granulation, pelletation, dry powder blend, fluid bed granulation, encapsulation, or compactation

25. The use of a composition according to any of claims 1 to 24 for the preparation of a pharmaceutical for female contraception, for hormone replacement therapy, for the treatment of menopausal, pre-menopausal, and/or post-menopausal symptoms, for the treatment of acne or for the treatment of PMDD.

26. A pharmaceutical particulate formulation of a composition, said composition comprising an inclusion complex between a cyclodextrin, or derivative thereof,
wherein the total amount of oxidant in said formulation is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen.

27. A pharmaceutical particulate formulation of a composition, said composition comprising an inclusion complex between a cyclodextrin, or derivative thereof,
wherein the total amount of oxidant in said formulation is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen

28. A pharmaceutical particulate formulation of a composition, said composition comprising an inclusion complex between a cyclodextrin, or derivative thereof,
wherein the total amount of oxidant in said formulation is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen.

29. A formulation according to claim 26, wherein the total amount of oxidant in said formulation is such that the decrease in total estrogen content, as measured at the 12-month mark at 40°C and 75% relative humidity (RH), is at least 0.1% less than, such as at least 0.2, 0.3, 0.4 or 0.5% less than the decrease in estrogen content in pharmaceutical formulations of compositions not comprising an inclusion complex between a cyclodextrin, or derivative thereof, and an estrogen, as measured at the same time mark under the same conditions.

30. A formulation according to claim 27, wherein the total amount of oxidant in said formulation is such that the decrease in total estrogen content, as measured at 3-month mark at 60°C and 75% relative humidity (RH) is at least 0.1% less than, such as at least 0.2, 0.3, 0.4 or 0.5% less than the decrease in estrogen content in pharmaceutical formulations of compositions not comprising an inclusion complex between a cyclodextrin, or derivative thereof, and an estrogen as measured at the same time mark under the same conditions.

31. A pharmaceutical particulate formulation of a composition, said composition comprising an inclusion complex between a cyclodextrin, or derivative thereof,
wherein the amount of polyvinylpyrrolidone in said formulation is such that said estrogen has a 12-month stability at 40°C and 75% relative humidity (RH) corresponding to a decrease in the content of said estrogen of at the most 10% w/w, such as at the most 5, 4, or 3% w/w, based on the total content of said estrogen.

32. A pharmaceutical particulate formulation of a composition, said composition comprising an inclusion complex between a cyclodextrin, or derivative thereof,
wherein the amount of polyvinylpyrrolidone in said formulation is such that said estrogen has a 3-month stability at 60°C and 75% relative humidity (RH) corresponding to a decrease in content of said estrogen of at the most 15% w/w, such as at the most 10, 9, 8, 7, or 6% w/w, based on the total content of said estrogen.

33. A pharmaceutical particulate formulation of a composition, said composition comprising an inclusion complex between a cyclodextrin, or derivative thereof,
wherein the amount of polyvinylpyrrolidone in said formulation is such that said ethinyl estradiol has a 12-month stability at 25°C and 60% relative humidity (RH) such that the sum product of the degradation products 6-α-hydroxy-ethinyl estradiol, 6-β-hydroxy-ethinyl estradiol, 6-keto- ethinyl estradiol and Δ9,11-ethinyl estradiol of said ethinyl estradiol totals at the most 0.8%, such as at the most 0.7% and 0.6%, based on the total content of estrogen.

34. A formulation according to any of claims 26 to 33 **characterised in that** it is a polyvinylpyrrolidone-free formulation.

35. A formulation according to any of claims 26 to 34, further comprising an anti-oxidant.

36. A formulation according to any of claims 26 to 35, comprising (wt/wt)
i) 0.002-1% ethinyl estradiol (micronised);
ii) 10-99%, such as 45-60% lactose monohydrate;
iii) 1-80%, such as 1-35% corn starch;
iv) 0-90%, such as 0-35% cellulose (microcrystallised);
v) 0.05-5% magnesium stearate and
vi) 0-5% colloidal silicon dioxide.

37. A formulation according to any of claims 26 to 35, comprising (wt/wt)
i) 0.1-15% drospirenone (micronised);
ii) 0.002-1% ethinyl estradiol (micronised);
iii) 10-99% such as 45-60% lactose monohydrate;
iv) 1-80%, such as 1-35% corn starch
v) 0-90%, such as 0-35% cellulose (microcrystallised);
vi) 0.05-5% magnesium stearate and
vii) 0-5% colloidal silicon dioxide.

38. A formulation according to any of claims 26 to 37 prepared by a method involving conventional granulation, pelletation, dry powder blend, fluid bed granulation, encapsulation, or compactation

39. A formulation according to any of claims 26 to 38 in unit dosage form.

40. A formulation according to any of claims 26 to 39 adapted for the preparation of solid dosage forms or dispersion forms.

41. A formulation according to any of claims 26 to 39 adapted to the preparation of tablets, capsules or sachets.

42. A composition according to any of claims 1 to 24 adapted to be administered by oral, parenteral, mucosal, nasal, vaginal or topical administration.

43. A method for improving the stability of ethinyl estradiol in a pharmaceutical composition comprising the step of forming an inclusion complex between said ethinyl estradiol and cyclodextrin.

44. A method of improving the stability of ethinyl estradiol in a pharmaceutical formulation comprising the step of a) complexing said ethinyl estradiol with cyclodextrin, and optionally of b) limiting the polyvinylpyrrolidone content to less than 5%, such as less than 4%, such as less than 3%, such as less than 2%, such as less than 1%, such as providing a substantially polyvinylpyrrolidone-free formulation.

45. A process for the preparation of a formulation according to any of claims 26 to 41.
